# EUROPEAN PATENT APPLICATION

(11) **EP 2 730 291 A1**
(43) Date of publication of application: **14.05.2014**
(21) Application number: 13192254.4
(22) Date of filing: 11.11.2013
(51) Int. Cl.: A61K 47/06, A61K 47/34

(54) **Internal Tamponade Composition**

(30) Priority: 09.11.2012 EP 12191962
(71) Applicant: FLUORON GMBH, 89077 Ulm (DE)
(72) Inventor: Kugler, Wilfried, 37139 Adelebsen (DE); Häring, Tim, 89284 Pfaffenhofen a.d. Roth (DE); Hagedorn, Nadine, 89134 Blaustein (DE); Theisinger, Bernhard, 68229 Mannheim (DE)
(74) Representative: Schiweck, Weinzierl & Koch

(57) **Abstract**

The present invention relates to a composition for use in the treatment of retinal detachment. In particular, the composition comprises a mixture of at least two oils and one or more semifluorinated alkanes for use as temporary tamponades in the treatment of retinal detachment.

## Description

The present invention relates to a composition for use in the treatment of retinal detachment. In particular, the composition comprises a mixture of at least two oils, preferably silicone oils, and one or more semifluorinated alkanes for use as temporary tamponades in the treatment of retinal detachment.

Retinal detachment is the separation of the neurosensory retina from its underlying pigment epithelium. Untreated, retinal detachment can result in permanent vision loss or blindness. Retinal detachment is caused by traction of the vitreous upon the retina. The traction can be 'dynamic', caused by eye movements and thus relative movement of the vitreous and the retina; or 'static', due to contraction of membranes on the surface of the retina. Retinal detachments are associated with myopia, pseudophakia, trauma and diabetes; it is often the common pathway leading to blindness in a host of ophthalmic eye diseases.

Where a retinal detachment is associated with retinal breaks (also referred to as perforations, holes or tears), fluid gains access from the vitreous cavity to the subretinal space. This form of retinal detachment is referred to as 'Rhegmatogenous'. There are only two effective means of closing retinal breaks. The first involves the application of explants outside the eye in order to buckle the sclera (such as described in U.S. Pat. No. 6,547,714). The second involves the use of intraocular tamponades. Intraocular tamponades are agents injected into the vitreous cavity to occlude retinal breaks. They are fluids that are immiscible with water and form an interface with it. As an alternative to a fluid, a gas can be used such as air, sulphur hexafluoride (SF6) or octafluoropropane (C3F8). These gases can be used undiluted in small volumes or mixed with air and totally fill the vitreous cavity. The liquids include perfluorocarbon liquids, semifluorinated alkanes or alkenes and silicone oil. Of these, only silicone oil can be tolerated in the eye for more than a few weeks. Prolonged use of any of the other liquids will give rise to retinal toxicity as demonstrated by inflammatory reaction or by histological changes.

Whilst these methods of treatment can be successful, they often require multiple treatments and there is always a danger of physical damage to the retina itself. Furthermore, there are also other dangers associated with these procedures such as the risk of infection, bleeding, high pressure inside the eye and the possibility of cataract development and these procedures often require a second operation to be undertaken.

Retinal detachments can also be treated by means of pneumatic retinopexy, whereby a gas bubble is injected into the vitreous space so as to help push the retinal tear back against the wall of the eye. This method can also be used in conjunction with the laser and cryo-surgical techniques if required. The gases preferred for such operations are commonly either hexafluoroethane (C2F6), octafluoropropane (C3F8) or sulphur hexafluoride (SF6), which when mixed with sterile air have the properties of remaining in the eye for extended periods of time. RU2235527 discloses a number of other gases that may also be used in conjunction with this technique.

Eventually, the gas is replaced by the eye's own natural fluid, although there have been recent concerns over the toxicology of compositions that are fluorine based.

Another method of treatment involves a vitrectomy whereby all or part of the vitreous gel is removed from the eye and replaced with a tamponade agent, such as a perfluorocarbon liquid, silicone oil or a gas (using a similar gaseous composition as described above). When a gas is used, the eye is allowed to fill with the body's own fluid over time. In this technique, a small incision is made into the sclera and the vitreous gel is removed by means of a small cutting device. As the vitreous gel is removed, a saline solution is used to maintain the pressure by a continuous infusion. Afterwards, an air/gas mixture is injected. Alternatively, perfluorocarbon liquids, semifluorinated alkanes or alkenes and more commonly silicone oils are injected as a tamponade agent. The tamponade agent is the immiscible fluid that occludes retinal breaks because of its interfacial tension and its buoyancy. The tamponade material is therefore intended to close the retinal tear and reoppose the retina on the underlying pigment epithelium. These latter tamponades must however be removed later. While perfluorocarbons are most frequently used as intraoperative tamponades, silicone oils are commonly used as long term tamponades.

Due to their lower specific density as compared with water, silicone oils are capable of exerting their tamponade effect in particular on retinal detachments associated with retinal breaks in the superior region of the retina. Due to their buoyancy, silicone oils are less efficient in the inferior region of the retina where, however, post-operative renewed detachment of the retina correlated with proliferative vitreoretinopathy (PVR) may occur to an increased extent. In this lower region, growth factors are concentrated in a PVR-stimulating environment, thus promoting renewed retinal detachment. Heavy silicone oils such as Densiron 68 or Oxane HD having a higher density than water displace this environment from the lower, inferior region and seal retinal breaks occurring therein in a more efficient manner.

For a number of years, 1,000 mPas silicone oil (i.e. a silicone oil with a viscosity of 1,000 mPas) has been used clinically as a tamponade agent, but after a period of time within the eye the oil has a tendency to emulsify. The emulsification causes a number of specific problems; firstly the emulsified droplets can stimulate adverse biological responses including inflammatory reactions. Secondly, blocking of fluid to the eye decreases aqueous outflow and can cause raised intraocular pressure and glaucoma. Thirdly, reduction of vision by opacification of the fluid within the eye, and lastly the emulsion cannot tamponade the retina. To reduce the risk of emulsification, the more viscous 5,000 mPas silicone oil is favoured by a number of clinicians.

The tendency to emulsify is increased by the presence of low molecular weight component silicone oil molecules. Impurities such as cyclical forms of siloxanes also contribute to the tendency of the silicone oil to disperse. The lower the overall molecular weight of the silicone oil and the lower the viscosity the less shear force is required to disperse the silicone oil. Previous attempts to reduce the tendency to dispersion include the manufacture of the so-called highly purified silicone oil, concentrating on removal of the low molecular weight components. Some manufacturers claim to aim for a single molecular weight silicone oil. Others approach the problem by increasing the viscosity and the purity of the medical product, such that 5,000 mPas oil has found favour amongst many surgeons.

As there is an increasing tendency to use small cannula sizes (e.g. 23 gauge or smaller) to inject fluids into the vitreous body, the viscosity of the silicone oils gains more importance. Whilst highly viscous oils can be injected under increasingly high pressures through small incisions into the eye, the same cannot be said of removal. The maximum suction force that can be generated by a suction pump is one atmosphere pressure, and this often results in a very low flow and thus a long time for the extraction of silicone oil. This often requires a compromise involving the use of a larger bore instrument and cannula which in turn requires the use of larger incisions into the eye. Such large incisions are undesirable surgically. They weaken the eye and increase the chances of collateral damage such as vitreous incarceration or entry site related tears. Hypotony can also follow once the oil has been removed as the balanced salt solution can now flow out relatively unimpeded via an enlarged incision. In all instances it is preferable to have small incisions with the minimal amount of invasive surgery.

Therefore many attempts have been undertaken to find a tamponade that has lower emulsification tendencies whilst still easily injectable. In several documents the use of 1,000 mPas silicone oil in admixture with 2,500,000 mPas silicone oil was outlined as ideal, preferably in a range from 95%/5% to 90%/10% (e.g. Increasing the extensional viscosity of silicone oil reduces the tendency for emulsification. Williams R. L et al., Retina , 2010; 30(2): 300-304). An admixture with a higher content of 2,500,000 mPas silicone oil was found to be undesirable (Development of Emulsification-Resistant Silicone Oils: Can we go beyond 2000mPas Silicone Oil? Caramoy A. et al., IOVS, 2011; 52(8): 5432-5436).

At present Densiron 68 (5,000 mPas silicone oil (69.5%) in admixture with F6H8 (Perfluorohexyloctane) (30.5%)) and Siluron 2000 (1,000 mPas silicone oil (95%) in admixture with 2,500,000 mPas silicone oil (5%)) are used as tamponades in the treatment of retinal detachment. Also, a long-term tamponade product, called "Xtra" is used that is composed of 90% 1,000 mPas silicone oil and 10% 2,500,00 mPas silicone oil with a viscosity of 4100 - 4800 mPas.

It is therefore an object of the present invention to address one or more problems associated with the prior art procedures and in particular to provide a tamponade agent that can be effectively used in the treatment of retinal detachment. Furthermore, it is an object of the present invention to provide a tamponade agent that resists emulsification or substantially prevents emulsification from occurring and is well tolerated in the eye. It is also an object of the present invention to provide a fluid tamponade agent that is user friendly, has a good injectability with a relatively low shear viscosity and a high biocompatibility.

Accordingly, heavier than water tamponade agents have been developed to support the inferior retina, thereby improving the currently available heavy tamponades Oxane HD and Densiron 68. One significant disadvantage using current heavy tamponades is their emulsification tendency. Since current heavy tamponades are a mixture of semifluorinated alkanes and polydimthylsiloxane, they emulsify more than standard silicone oil.

A mixture of first and second oil which differ from each other in their viscosity and the addition of a semifluorinated alkane increase the fluid viscoelasticity and show an increased emulsification resistance in comparison to state of the art heavy tamponades being composed of a silicone oil and a semi-fluorinated alkane. In addition, the compositions have a good injectability.

The present invention can be summarized by the following items:
1. A composition comprising a mixture of at least two oils (first and second oil) and one or more semifluorinated alkanes.
2. The composition of item 1 which is for use in the treatment of a detached retina.
3. The composition of any one of the preceding items, wherein a first oil has a viscosity of about 5,000 mPas or less and is selected from one or more of a silicone oil, a fluorosilicone oil, a silicone-fluorosilicone copolymer oil, a silicone-phenylsilicone copolymer oil, a silicone oil derivative or a mixture thereof.
4. The composition of any one of the preceding items, wherein a second oil has a viscosity in the range of about 10,000-25,000,000 mPas and is selected from one or more of a silicone oil, a fluorosilicone oil, a silicone-fluorosilicone copolymer oil, a silicone-phenylsilicone copolymer oil, a silicone oil derivative or a mixture thereof.
5. The composition of any one of the preceding items, wherein the one or more semifluorinated alkanes are selected from one or more of perfluorobutylpentane (F4H5), perfluorobutylhexane (F4H6), perfluorobutyloctane (F4H8), perfluorohexylhexane (F6H6), perfluorohexyloctane (F6H8) or a mixture thereof.
6. The composition of any one of the preceding items, wherein the first oil is present in the mixture of the first and the second oil in a quantity in the range of about 75-92.5% w/w.
7. The composition of any one of the preceding items, wherein the second oil is present in the mixture of the first and the second oil in a quantity in the range of about 7.5-25% w/w
8. The composition of any one of the preceding items, wherein the one or more semifluorinated alkanes are present in the composition in a quantity in the range of about 20-40% w/w.
9. The composition of any one of the preceding items, wherein the composition optionally comprises additives selected from one or more of emulsification inhibitors, steroids, non-steroidal anti-inflammatory agents, antibiotics or a mixture thereof.
10. The composition of any one of the preceding items, wherein the composition has a dynamic viscosity in a range of about 900 mPas to about 5,500 mPas and a density in the range of about 1.010 g/cm³ to about 2.500g/cm³.
11. The composition of any one of the preceding items, wherein the first oil is a silicone oil having a viscosity of about 1,000 mPas and the second oil is a silicone oil having a viscosity of about 2,500,000 mPas and the used one or more semifluorinated alkane is perfluorobutylpentane (F4H5) and/or perfluorohexyloctane (F6H8).
12. The composition of any one of the preceding items, wherein the first oil is present in the mixture of the first and the second oil in a quantity in the range of about 84-86% or 85-87.5% w/w.
13. The composition of any one of the preceding items, wherein the second oil is present in the mixture of the first and the second oil in a quantity in the range of about 12.5-15% or 14-16% w/w.
14. The composition of any one of the preceding items, wherein the one or more semifluorinated alkanes are present in the composition in a quantity in the range of about 36-38% w/w.
15. The composition of any one of the preceding items, wherein the first oil is present in the mixture of the first and the second oil at about 85% w/w, the second oil is present in the mixture of the first and the second oil at about 15% w/w and the semifluorinated alkanes are present in the composition at about 37 % w/w.
16. The composition of item 15, wherein the first oil is a silicone oil and the second oil is a silicone oil.
17. The composition of item 15 or 16, wherein the first oil has a viscosity of 1,000 mPas or less and the second oil has a viscosity of 2,500,000 mPas.
18. The composition of any one of items 15 to 17, wherein the one or more semifluorinated alkanes is perfluorobutylpentane (F4H5).
19. A method of producing a composition for use in the treatment of a detached retina, comprising mixing at least two oils and one or more semifluorinated alkanes to produce a composition of any one of items 1 to 18.
20. A kit of parts comprising the composition of any one of items 1 to 18 in a vial or syringe, one or more of cannulas and/or tubing, and instructions for use.
21. The composition of any one of items 1 to 18 for use as temporary tamponade in the treatment of retinal detachment.
22. The composition of item 21, wherein retinal detachment includes ruptures in the retina, detachment because of a trauma, detachment with vitreoretinal proliferation and detachment in case of proliferative diabetic retinopathy.
23. A method of treating retinal detachment in a subject in need thereof, comprising administering a composition of any one of items 1 to 18 into the vitreous body of the eye of said subject.
24. Use of a composition of any one of items 1 to 18 for the preparation of a composition for treating retinal detachment.
25. Use of a composition of any one of items 1 to 18 as temporary tamponade.
26. A method of producing a composition of any one of items 1 to 18 comprising mixing a first oil, a second oil and one or more semifluorinated alkanes as defined in any one of items 1 to 18.

In accordance with the present invention, there is provided a composition for use in the treatment of a detached retina, comprising at least two oils and one or more semifluorinated alkanes. The at least two oils, i.e. a first and second oil are in the form of a mixture, i.e., a mixture between at least the first and second oil. Generally, the first and second oil differ from each other at least in their viscosity, for example, the first oil may have a viscosity of about 5,000 mPas or less, such as about 3,000 mPas, about 2,000 mPas or 1,000 mPas, while the second oil may have a viscosity of about 5,000 mPas or more, such as 10,000-25,000,000 mPas, about 30,000-20,000,000 mPas, or about 50,000-5,000,000 mPas.

"At least" in the context of the mixture of at least two oils means that the mixture of the first and second oil may comprise one or more further oils, e.g., a third and/or fourth, etc. oil. Accordingly, it is preferred that a mixture between a first and second oil is prepared and said mixture is then mixed with one or more semifluorinated alkanes or vice versa. By way of illustration, the first and second oil constitute together 100% w/w. A portion "x" of said 100% w/w is then mixed with a portion "y" of one or more semifluorinated alkanes in order to obtain a composition having x% w/w and y% w/w, whereby x% w/w plus y% w/w is 100% w/w.
The composition of the present invention is composed of at least two parts, i.e., a mixture of at least two oils (at least a first and second oil) and one or more semifluorinated alkanes. "At least" in the context of the composition means that the composition of the present invention may contain in addition to the mixture of at least two oils and one or more semifluorinated alkanes one or more further parts or ingredients, such as other liquids, nanoparticles, or medicaments.

The composition of the present invention provides for an oil-mixture which can be used to replace part or all of the vitreous gel/humour in the posterior part of the eye, is highly tolerated and does not have a propensity to emulsify. The resultant solution has ideally greater resistance to emulsification due to increased extensional viscosity while exhibiting a high biocompatibility. "Detached retina" also includes ruptures in the retina, detachment because of a trauma, detachment with vitreoretinal proliferation and detachment in case of proliferative diabetic retinopathy, respectively.

Extensional viscosity can be understood by considering a dilute solution of a high molecular weight linear polymer, where the properties of the solution are governed by the isolated individual polymer coils. At low rates of extension of the solution, the polymer chains are in a loosely spherical configuration and the extensional viscosity is low. At higher extension rates, however, the polymer chains unwind and elongate in line with the direction of extension, presenting a resistance to the applied extensional flow. At a critical extensional flow rate, the polymer chains unwind into a long stretched string. This is known as the coil-stretch transition and results in a large increase in resistance to the applied extension and hence an extensional viscosity that can be many times higher than the equivalent viscosity in shear flow.

The process of emulsification can be considered as the oscillation of shearing of an oil/water interface under an external force which leads to the pulling out of filaments of one liquid into another. These filaments subsequently thin on extension then snap resulting in the formation of satellite droplets that, subject to suitable emulsification stabilizers being present, will persist in the continuous phase. By increasing the extensional viscosity of the oil phase, filament breakage is inhibited and therefore this increase in extensional viscosity hinders satellite droplet formation.

The first oil used in the composition of the present invention may be a low viscosity oil such as oils currently used in procedures for the treatment of retinal detachment. Preferably, the oil may have a viscosity of about 5,000 mPas or less. Preferably, the viscosity of the oil is up to about 3,000 mPas. Preferably, the oil has a viscosity of up to about 2,000 mPas. Preferably, the oil has a viscosity of about 1,000 mPas. Viscosity is preferably measured at room temperature, more preferably at a temperature of 25°C and normal pressure, for example, with a capillary viscometer (viscosimeter), such as an Ubbelohde viscosimeter.

The first oil used in accordance with the present invention may be one which is currently used during the treatment of retinal detachment. It will be evident to the skilled addressee that the term "oil" will also encompass oils which have yet to be developed. Preferably, the oil is selected from one or more of the following: a silicone oil, a fluorosilicone oil, a silicone-fluorosilicone copolymer oil, a silicone-phenylsilicone copolymer oil, a silicone oil derivative or a mixture thereof. More preferably the first oil used in accordance with the present invention is a silicone oil. Preferably, the first oil is present in the mixture in a quantity in the range of about 70-95% w/w, such as 75-92.5% w/w, 85-90% w/w or 85-92.5% w/w. In the alternative, but also preferred, the first oil is present in the mixture in a quantity of about 70% w/w, 75% w/w , 80% w/w , 85% w/w, 90% w/w, 95% w/w or more, with 85% w/w being preferred. In another preferred alternative, the first oil is present in the mixture in a quantity in the range of about 80-92.5% w/w, 85-87.5% w/w or 84-86% w/w, such as 80%, 85%, 87%, 88%, 89%, 89.5%, 90% or 92.5% w/w.

According to the invention preferably flowable silicones are used as silicone oils, in particular siloxanes, which satisfy both the required properties. Methylsiloxanes and their polymers and dimethylsiloxanes and their polymers are highly suitable. The silicones can be both unsubstituted and substituted. Substitution is understood as meaning the replacement of a hydrogen atom bonded to a silicon atom by a different atom or a molecular group. Common substituents are straight-chained, branched or cyclic alkyl groups having from 1 to 18 carbon atoms or aryl groups having from 6 to 15 carbon atoms. The alkyl or aryl groups can themselves be substituted. The substituents can be present in blocks or distributed over the siloxane chain, and the amount thereof can be adjusted in a known manner according to the desired properties.

Preferred silicone oils according to the present invention also include inter alia: n-polydimethylsiloxanes, iso-polydimethylsiloxanes, aryl-substituted PDMS, such as phenyl-polydimethylsiloxanes, diphenyl-polydimethylsiloxanes, polyphenyl-polydimethylsiloxanes, alkyl-substituted PDMS, such as methyl-polydimethylsiloxanes, ethyl-polydimethylsiloxanes, propyl-polydimethylsiloxanes and the like, PDMS having other substituents, such as fluoroalkyl-substituted PDMS. This list is intended to give examples of suitable silicone oils according to the present invention, without being limited thereto. Examples of other suitable silicone oils are bis-diphenylethyl disiloxane, bisphenyl hexamethicone, capryl dimethicone, caprylyl dimethicone, caprylyl methicone, dimethicone, disiloxanes, hexyl dimethicone, hexyl methicone, lauryl dimethicone, lauryl methicone, methicone, methyl trimethicone, phenylethyl dimethicone, phenylethyl disiloxane, phenylpropyl ethyl methicone, phenylpropyl trimethicone, phenyl trimethicone, trifluoropropyl dimethicone, trifluoropropyl methicone, trisiloxanes, etc.

The second oil used in accordance with the present invention will have a high molecular weight and will be soluble/miscible with the first oil. More preferably, the second oil will be soluble/miscible with a silicone oil, a fluorosilicone oil, a silicone-fluorosilicone copolymer oil, a silicone-phenylsilicone copolymer oil, a silicone oil derivative or a mixture thereof. It will be apparent that a number of oils may be suitable for increasing the extensional viscosity of the first oil. Oils that will not impair the optical characteristics of the first oil will be most desirable. Furthermore, the high molecular weight oils will preferably be those miscible with or soluble in silicone tamponade oils.

The second oil has preferably a viscosity of at least about 5,000 mPas. Preferably, the viscosity of the oil is in the range of about 10,000-25,000,000 mPas, more preferably in the range of about 30,000-20,000,000 mPas, and yet more preferably in the range of about 50,000-5,000,000 mPas. Most preferably the second oil has a viscosity of about 2,500,000 mPas.

The second oil used in accordance with the present invention may be one which is currently used during the treatment of retinal detachment. It will be evident to the skilled addressee that the term "oil" will also encompass oils which have yet to be developed. Preferably, the oil is selected from one or more of the following: a silicone oil, a fluorosilicone oil, a silicone-fluorosilicone copolymer oil, a silicone-phenylsilicone copolymer oil, a silicone oil derivative or a mixture thereof. More preferably the second oil used in accordance with the present invention is a silicone oil.

The second oil may be a mixture of two or more oils if required. For example, the second oil may be a mixture of two high viscosity silicone oils, such as those having viscosities in the ranges as described above.

The composition optionally comprise further oils which may be selected from one or more of the following: a silicone oil, a fluorosilicone oil, a silicone-fluorosilicone copolymer oil, a silicone-phenylsilicone copolymer oil, a silicone oil derivative or a mixture thereof.

It will be apparent to one skilled in the art that the quantity of the second oil relative to the quantity of the first oil will depend upon a number of factors, not least the particular molecular weights and/or viscosities of the first and the second oil. For example, a small quantity of a larger molecular weight second oil may produce the same increase in extensional viscosity of the first oil as a larger quantity of a smaller molecular weight second oil. It is preferable that the quantity of second oil is relatively low in the composition so that the additive does not detrimentally affect the shear viscosity of the oil. Controlling the shear viscosity of the oil is important so that the composition can be inserted into the eye using the smallest incision as possible. Preferably, the second oil is present in the mixture of the first and the second oil in a quantity in the range of about 5-30% w/w, such as 7.5-25 % w/w, 7.5-15% w/w or 10-15% w/w. In the alternative, but also preferred, the second oil is present in the mixture in a quantity of about 10% w/w or more. In a yet further preferred alternative, the second oil is present in the mixture in a quantity in the range of about 7.5-20% w/w, 12.5-15% or 14-16% w/w, such as 7.5% w/w, 10% w/w, 10.5% w/w, 11 % w/w, 12% w/w, 13% w/w, 15% w/w or 20% w/w, with 15% w/w being preferred. Other alternatively preferred amounts of the second oil in the mixture are 5% w/w, 10% w/w, 15% w/w, 20% w/w, 25% % w/w or 30% w/w.

The one or more semifluorinated alkanes used in the composition of the present invention may be selected from perfluorobutylpentane (F4H5), perfluorobutylhexan (F4H6), perfluorobutyloctane (F4H8), perfluorohexylhexane (F6H6), perfluorohexyloctane (F6H8) or a mixture thereof. Preferably the one or more semifluorinated alkanes are selected from perfluorobutylpentane (F4H5), perfluorohexyloctane (F6H8) or a mixture thereof. Most preferably the semifluorinated alkane used in the composition of the present invention is perfluorobutylpentane (F4H5).

The quantities of the one or more semifluorinated alkanes in the composition of the present invention also affect the physical properties, like extensional viscosity, shear viscosity, emulsion tendency, refraction index, density and biocompatibility of the composition of the present invention.

Preferably, the one or more semifluorinated alkanes are present in the composition a quantity in the range of about 10-40% w/w or 20-40% w/w or 30-40% w/w of the composition, which means, e.g. about 20-40% w/w of the one or more semifluorinated alkanes in admixture with about 60-80% w/w of the at least two oils. More preferably, the one or more semifluorinated alkanes are present in the composition in a quantity in the range of about 30-40% w/w. Even more preferably, the one or more semifluorinated alkanes are present in the composition in a quantity in the range of about 33-39% w/w including in a quantity in the range of about 36-38% w/w. or in a quantity of 37% w/w. As explained above, in addition to the one or more semifluorinated alkanes, the composition of the present invention comprises a mixture of at least two oils. Hence, apart from the one or more semifluorinated alkanes, the other part of the composition of the present invention that renders the composition to have 100% w/w is constituted by the mixture of at least two oils. Connecting to the above calculation outlined for the mixture, a composition of the present invention is composed of (x+y)% w/w of a mixture of at least two oils and z% w/w of one or more semifluorinated alkanes, i.e., (x+y)% w/w mixture plus z% w/w semifluorinated alkanes. For example, when the one or more semifluorinated alkanes are present in the composition in the range of about 20-40% w/w, then the mixture of at least two oils is present in the range from about 60-80% w/w.
However, if the composition of the present invention comprises, apart from the one or more semifluorinated alkanes (z% w/w) and the mixture of at least two oils ((x+y)% w/w) a further liquid, then either the quantity of the one or more semifluorinated alkanes and/or the mixture of at least two oils is reduced accordingly such that the composition has its 100% w/w. Accordingly, either the quantity of the one or more semifluorinated alkanes or the quantity of the mixture of at least two oils is reduced or both the quantity of the one or more semifluorinated alkanes and the quantity of the mixture of at least two oils is reduced.

When used herein, the term "about" is understood to mean that there can be variation in the described formulation that can be to 5%, 10%, 15% or up to and including 20% of the given value. For example, if an oil such as a silicone oil has a viscosity of 5,000 mPas, it means that a variation of, e.g., 10% includes an oil with a viscosity of 5,500 mPas and a variation of, e.g. 20% includes an oil with a viscosity of 6,000 mPas. For example, if a formulation has about 5 mg/mL of a compound, this is understood to mean that a formulation can have between 3 to 7 mg/mL, more preferably between 4 to 6 mg/mL. Thus, as used herein, a concentration interval which is defined as "X to Y" or "X-Y" equates with an interval which is defined as "between X and Y". Both intervals specifically include the upper limit and also the lower limit. This means that for example an interval "5-10% w/w" or "between 5% and 10% w/w" includes a concentration of 5, 6, 7, 8, 9, and/or 10% w/w.

The composition of the present invention may further comprise additives like emulsification inhibitors (e.g. hydrophobic additives or detergents) or pharmacologically active agents, like steroids e.g. corticosteroids like glucocorticoids (e.g. dexamethasone), fluocinolone acetonide or triamcinolone acetonide; other non-steroidal anti-inflammatory agents like acetyl-salicylic acid, phenylbutazone or nabumetone; or antibiotics like vancomycin and ceftazidime; but not limited thereon.

The composition of the present invention exhibits a dynamic viscosity in the range of about 900 mPas to about 5,500 mPas, measured by a Ubbelohde-viscosimeter. Preferably the dynamic viscosity is in the range of about 1,100 mPas to about 4,500 mPas. More preferably the dynamic viscosity is in the range of about 1,300 mPas to about 2,400 mPas. Even more preferably the dynamic viscosity is in the range of about 1,350 mPas to about 1,600 mPas, with adynamic viscosity of about 1,400 mPas being preferred.

The dynamic viscosity, measured in Pa·s, is calculated by multiplying the kinematic viscosity of a liquid with its density.

Moreover the composition of the present invention exhibits a density in the range of about 1.010 g/cm³ to about 2.500g/cm³, measured, for example, by a DA 100M densitometer (Mettler-Toledo). Preferably the density is in the range of about 1.020 g/cm³ to about 1.100, or in the range of about 1.040 g/cm³ to about 1.080 g/cm³, or in the range of about 1.050 g/cm³ to about 1.070 g/cm³ , with a density of in the range of about 1.060 g/cm³ and about 1.065 g/cm³.being preferred. Density is preferably measured at room temperature, more preferably at a temperature of 25°C and under normal pressure.

The composition of the present invention may, on decision of the surgeon, be applied and removed with 20 to 30 gauge instruments (e.g. cannulas), preferably with 20 to 25 gauge instruments, more preferably with 23 gauge instruments.

In accordance with another aspect of the present invention, there is provided a method of producing a composition for use in the treatment of a detached retina, comprising mixing at least two oils and one or more semifluorinated alkanes. The method may be used to produce a composition as described above.

A yet further aspect of the present invention provides for a kit of parts for applying/injecting and/or removing a composition of the present invention in the treatment of a detached retina comprising a composition of the present invention (e.g. in a vial or syringe), together with one or more of cannulas, tubing and/or other useful equipment to inject or remove the composition of the present invention, and instructions for use.

In accordance with yet a further aspect of the present invention, there is provided a composition for use in the treatment of a detached retina, comprising a mixture of at least two oils and one or more semifluorinated alkanes.

The present invention also provides a method of treating retinal detachment in a subject in need thereof, comprising administering a composition of the present invention into the vitreous body of the eye of said subject.

Also, the present invention provides a use of a composition as described herein for (use in a method of) treating retinal detachment.

Furthermore, provided herein is a use of a composition as described herein as temporary tamponade.

The present invention moreover provides a method of producing a composition as described herein comprising mixing a first oil, a second oil and one or more semifluorinated alkanes as described herein.

It should be understood that the inventions disclosed herein are not limited to particular methodology, protocols, or reagents, as such can vary.

## Claims

1. A composition comprising a mixture of at least two oils (first and second oil) and one or more semifluorinated alkanes.

2. The composition of claim 1 which is for use in the treatment of a detached retina.

3. The composition as claimed in any preceding claim, wherein a first oil has a viscosity of about 5,000 mPas or less and is selected from one or more of a silicone oil, a fluorosilicone oil, a silicone-fluorosilicone copolymer oil, a silicone-phenylsilicone copolymer oil, a silicone oil derivative or a mixture thereof.

4. The composition as claimed in any preceding claim, wherein a second oil has a viscosity in the range of about 10,000-25,000,000 mPas and is selected from one or more of a silicone oil, a fluorosilicone oil, a silicone-fluorosilicone copolymer oil, a silicone-phenylsilicone copolymer oil, a silicone oil derivative or a mixture thereof.

5. The composition as claimed in any preceding claim, wherein the first oil is present in the mixture of the first and the second oil in a quantity in the range of about 70-95% w/w.

6. The composition as claimed in any preceding claim, wherein the second oil is present in the mixture of the first and the second oil in a quantity in the range of about 5-30% w/w

7. The composition as claimed in any preceding claim, wherein the one or more semifluorinated alkanes are present in the composition in a quantity in the range of about 20-40% w/w.

8. The composition as claimed in any preceding claim, wherein the one or more semifluorinated alkanes are selected from one or more of perfluorobutylpentane (F4H5), perfluorobutylhexan (F4H6), perfluorobutyloctane (F4H8), perfluorohexylhexane (F6H6), perfluorohexyloctane (F6H8) or a mixture thereof.

9. The composition as claimed in any preceding claim, wherein the first oil is a silicone oil having a viscosity of about 1,000 mPas and the second oil is a silicone oil having a viscosity of about 2,500,000 mPas and the used one or more semifluorinated alkane is perfluorobutylpentane (F4H5) and/or perfluorohexyloctane (F6H8).

10. The composition as claimed in any preceding claim, wherein the first oil is present in the mixture of the first and the second oil in a quantity in the range of about 85-87.5% w/w.

11. The composition as claimed in any preceding claim, wherein the second oil is present in the mixture of the first and the second oil in a quantity in the range of about 12.5-15% w/w.

12. The composition as claimed in any preceding claim, wherein the one or more semifluorinated alkanes are present in the composition in a quantity in the range of about 30-40% w/w.

13. The composition as claimed in any preceding claim, wherein the first oil is a silicone oil and the second oil is a silicone oil.

14. The composition as claimed in any preceding claim, wherein the one or more semifluorinated alkanes is perfluorobutylpentane (F4H5).

15. A kit of parts comprising the composition as claimed in any one of claims 1 to 14 in a vial or syringe, one or more of cannulas and/or tubing, and instructions for use.
